# EUROPEAN PATENT SPECIFICATION

(11) **EP 2 658 960 B1**
(45) Date of publication and mention of the grant of the patent: **18.10.2017**
(21) Application number: 12703432.0
(22) Date of filing: 02.01.2012
(51) Int. Cl.: C12N 1/06, C12N 1/12, C12N 9/20, C12N 9/24, C12P 23/00

(54) **IMPROVED PRODUCTION PROCESSES OF HAEMATOCOCCUS CELL COMPONENTS**
VERBESSERTES VERFAHREN ZUR HERSTELLUNG VON HAEMATOCOCCUS-ZELLBESTANDTEILEN
PROCÉDÉS DE PRODUCTION AMÉLIORÉS DE COMPOSANTS CELLULAIRES D'HAEMATOCOCCUS

(30) Priority: 31.12.2010 US 201061428943 P
(43) Date of publication of application: 06.11.2013
(73) Proprietor: Direvo Industrial Biotechnology GmbH, 50829 Köln (DE)
(72) Inventor: MILOS, Klaudija, 50829 Cologne (DE); STAMME, Claudia, 50829 Cologne (DE); WIENAND, Tanja, 50829 Cologne (DE); SVETLICHNY, Vitaly, 50829 Cologne (DE); SCHEIDIG, Andreas, 50829 Cologne (DE)
(74) Representative: Roth, Andy Stefan
(86) International application number: PCT/EP2012/000004
(87) International publication number: WO 2012/089842

(56) References cited:
- WO-A1-2010/104922
- WO-A2-2009/033071
- WO-A2-2010/149859
- US-A- 5 795 764
- US-A1- 2011 086 386
- DATABASE WPI Week 201041 Thomson Scientific, London, GB; AN 2010-B04763 XP002672762, -& KR 2009 0131991 A (NATURAL CHOICE CO LTD) 30 December 2009 (2009-12-30)

## Description

### FIELD OF THE DISCLOSURE

The technology disclosed herein relates to novel methods and compositions for the production processes of algae cell components from algae of the genus *Haematococcus.* The extractability of algae cellular components is improved and the process is optimized and accelerated by using the enzyme compositions according to the present invention.

### BACKGROUND

Generally, algae are grouped into two categories - microalgae and macroalgae - based on their morphology and size. As the name indicates, microalgae are microscopic photosynthetic organisms, many of which are unicellular. On the contrary, macroalgae which are commonly known as seaweeds, are composed of multiple cells which organize to structures resembling roots, stems, and leaves of higher plants.

Microalgae are thought to be one of the earliest life forms on earth and they are the fastest growing plants in the world. Since they can inhabit diverse ecological habitats ranging from freshwater, brackish water, or seawater, they are equipped to thrive in various extreme temperatures and pH conditions. These peculiarities make microalgae the most abundant organisms on earth.

Macroalgal in general, such as, *Laminaria*, *Saccorhiza*, *Alaria* are belonging to brown algal group and grows up to meters and their main reserved food material is laminarin and mannitol. The red algae such as *Gelidium amansii*, which is composed of cellulose, glucan and galactan, also can serve as a potential feedstock for bioconversion to ethanol.

In addition, algae yield unique biochemical substances which may be pharmacologically active, for example as viral inhibitors or cell division inhibitors, and substances which can serve as gelling and thickening agents and are used e.g. in the food industry. For example, carotenoids like astaxanthin, used as an antioxidant in human nutrition can be produced in green algae such as *Haematococcus pluvialis.* From the vast number of known marine and freshwater species of microalgae, only handfuls are currently of commercial significance. These include *Chlorella, Spirulina, Dunaliella* and *Haematococcus.*

Cultivation is the main way to generate biomass from microalgae. This has been done at industrial scale for many years. The most common production systems employed for algal cultivation are outdoor open ponds and enclosed photobioreactors (PBR). Production systems vary in terms of growth parameters control, contamination, water evaporation, productivity, downstream processing characteristics, capital and operational costs, etc.

Harvesting produces a slurry material with 2 - 7 % algal concentration. The next step is dewatering in order to get 15 to 25% concentration. This is usually achieved by pressing or centrifugation. These steps are normally integrated in the harvesting operation. Drying may be necessary for some applications. A very important issue in biomass treatment is the preservation of chemical quality.

The recovery of microalga biomass which generally requires one or more solid-liquid separation steps is a challenging phase of the algal biomass production process, and can account for 20-30% of the total costs of production. The processes involved include flocculation, filtration, flotation, and centrifugal sedimentation; some of which are highly energy consuming. Generally, microalgae harvesting is a two stage process, involving: (1) Bulk harvesting aimed at separation of biomass from the bulk suspension. The concentration factors for this operation are generally 100-800 times to reach 2-7% total solid matter. This will depend on the initial biomass concentration and technologies employed, including flocculation, flotation or gravity sedimentation. (2) Thickening-the aim is to concentrate the slurry through techniques such as centrifugation, filtration and ultrasonic aggregation, hence, are generally a more energy intensive step than bulk harvesting.

The harvested biomass slurry (typical 5-15% dry solid content) is perishable and must be processed rapidly after harvest; dehydration or drying is commonly used to extend the viability depending on the final product required. Methods that have been used include sun drying, low-pressure shelf drying, spray drying, drum drying, fluidized bed drying and freeze drying. Spray drying is commonly used for extraction of high value products, but it is relatively expensive and can cause significant deterioration of some algal pigments. Freeze drying is equally expensive, especially for large scale operations, but it eases extraction of oils. Intracellular elements such as oils are difficult to extract from wet biomass with solvents without cell disruption, but are extracted more easily from freeze dried biomass. The cost of drying is an important consideration in the processing of microalgal biomass powder for the food and feed industry and especially for the emerging biofuels industry.

Cell disruption is often required for recovering intracellular products from microalgae and performed to release intracellular products into the culture broth making them available for further separation processes. Cell disruption methods that have been used successfully include bead milling, highpressure homogenisers, autoclaving, ultrasonication super critical CO2 extraction and addition of hydrochloric acid, sodium hydroxide, or alkaline lysis. Solvents, like e.g. hexane, are widely used to extract metabolites such astastaxanthin, beta-carotene and fatty acids from algal biomass. Properties of the cell membrane play an important part in solvent extraction process. For example, the presence of a cell wall may prevent direct contact between the solvent and the cell membrane and impede the extraction.

One of the key challenges remains in development of aqueous extraction technologies as algal cells vary greatly in compositions between the species.

*Haematococcus pluvialis* is a freshwater species of Chlorophyta from the family Haematococcaceae. This species is well known for its high content of the strong antioxidant astaxanthin, which is important in aquaculture, various pharmaceuticals, and cosmetics. The high amount of astaxanthin is present in the resting cells, which are produced and rapidly accumulated when the environmental conditions become unfavorable for normal cell growth. The commercial production process is based on two distinct cultivation stages. The first is called the "Green Stage," which starts indoors with a single-cell colony of the microalga, and continues outdoors in solar-powered photobioreactors. The aim of this stage is to produce plenty of viable, unstressed "green" algal cells by normal cell-division process). The "Green Stage" provides optimal growth conditions in order to achieve maximal biomass production rate. The second cultivation stage is the "Red Stage", in which the algal cells synthesize and accumulate the pigment astaxanthin. This stage starts by subjecting the cells to severe stress conditions, mainly high radiation intensity and changes in growth media. As a result, the Haematococcus cells start to form cysts by producing thick cell walls, and to synthesize and accumulate astaxanthin in its esterified form.

Cultivating the algal culture in closed systems allows an environmentally controlled process with less biological and chemical contamination. Following the "Red" process, the level of astaxanthin in the "red cells" may reach up to ∼4% of their dry weight. The astaxanthin content of the "red cells" is correlated to the severity of the stress conditions, mainly to the light flux through the culture. In due time, the "red" culture is pumped to the down-processing area, where the cells are cracked (to render the pigment bioavailable), dried, and vacuum-packed. Haematococcus oleoresin is produced in an additional step, using the CO₂ Supercritical Fluid Extraction process. Increasingly, both consumers and regulatory agencies are requiring extracts that contain no residual solvents. U.S. Nutra of Eustis, FL, has the technology to extract Haematococcus with CO₂ and without any co-solvents.

However, enzymatic cell wall degradation is not widely practiced in the astaxanthin industry mainly because cell lysing enzymes have traditionally been cost prohibitive and the cyst cell walls of Haematococcus are known for its resistance to degradation. However, in the prior art enzymes do not retain some significant advantages over other methods of algal cell disruption, such as their degradation selectivity. Selectivity of enzymes is important in extraction of delicate and marginally stable chemicals. Haematococcus cell walls are more recalcitrant than the cell walls of other algae. Most microalgal cell walls contain cellulose but Haematococcus additional tri-laminar sheaths (TLS) containing algaenan, a substance known for its resistance to degradation (Allard et al., 2002, Versteegh and Blokker, 2004). As mentioned above, the Haematococcus cyst cell walls are highly resistant to degradation.

For example, the WO 2010/104922 A1 discloses the use of an enzyme composition comprising a lipase, cellulase and/or a protease for conditioning of an algal biomass in a method of astaxanthin extraction.

Furthermore, US 5 795 764 A, WO 2009/033071 A2 and WO 2010/149859 A2 describe the use of 40 mannanase to degrade algal material to improve extraction of components.

KR 2009 0131991 discloses a process for extracting astaxanthin from H. pluvialis using betaglucanase.

However, degrading these biopolymers using mechanical methods requires excess energy usage and multiple passes through disruption equipment lay a bead mill.

Therefore, there is a need for novel production methods for of cell components of algae belonging to the genus *Haematococcus* and for novel methods for improving the extractability these cell components.

### SUMMARY OF THE DISCLOSURE

The present invention is defined according to the claims and pertains to a method for improving the extractability of astaxanthin in form of the (3 S, 3 'S) isomer from an algae biomass comprising the steps of a) Subjecting the algae biomass to an enzyme composition comprising a mannanase, a laminarinase and a lipase,b) Extracting astaxanthin in form of the (3 S, 3 'S) isomer from the algae biomass, wherein the algae belongs to the species *Haematococcus pluvialis.*

In a further aspect the present disclosure pertains to methods for improving the extractability of an algae cellular component from an algae biomass comprising the steps of:
a) Subjecting the algae biomass to an enzyme composition comprising a mannanase and/or a lipases and/or a laminarinase
b) Extracting the cellular composition from the algae biomass,
wherein the algae belonging to the genus *Haematococcus.*

In a second aspect, the disclosure pertains to methods for producing an algae cell component by using algae belonging to the genus *Haematococcus* comprising the steps:
a) culturing the algae in a liquid medium,
b) harvesting the algae,
c) disrupting the algae cells by enzymatic treatment with an enzyme composition comprising a mannanase and/or a lipases and/or a laminarinase,
d) isolating the cell component.

In a third aspect, the present disclosure pertains to methods for producing a carotenoid by using a photoautotrophic algae belonging to the species *Haematococcus pluvialis* capable of producing an effective amount of a carotenoid comprising the steps of:
(a) culturing the algae cells under photoautotrophic conditions in a liquid medium,
(b) harvesting the algae cells,
(c) centrifuge the harvested cells for water removal
(d) enzymatic treatment of the algae cells or parts thereof with an enzyme composition comprising a mannanase and/or a lipases and/or a laminarinase
(e) isolating the carotenoid from the medium by extraction and/or by centrifugation.

In another aspect, the present disclosure pertains to the uses of n enzyme compositions comprising a mannanase, preferably a 1,4-beta- mannanase, more preferably an endo-1,4-beta- mannanase for the extraction of astaxanthin from *Haematococcus pluvialis.*

Further, the present disclosure pertains to the use of an enzyme composition comprising a lipase for the extraction of astaxanthin from *Haematococcus pluvialis.*

Furthermore, the present disclosure pertains to the use of an enzyme composition comprising a laminarinase for the extraction of astaxanthin from *Haematococcus pluvialis* and to the use of a mannanase, a lipase and/or a laminarinase, or mixtures thereof for the extraction of astaxanthin from *Haematococcus pluvialis.*

### BRIEF DESCRIPTION OF THE DRAWINGS

FIG. 1 shows the results of the treatment of *Haematococcus pluvialis* with different enzyme compositions
FIG. 2 shows the results of the treatment of *Haematococcus pluvialis* with an enzyme composition having mannanase activity

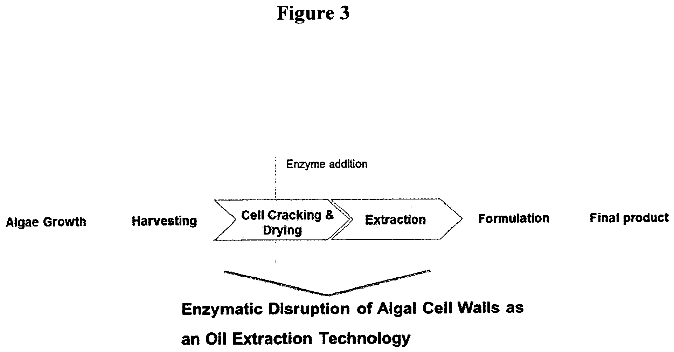

FIG.3 shows the general production process and extraction steps of microalgae
FIG.4 is a process flow chart of processing of *Haematococcus pluvialis* cells for astaxanthin extraction

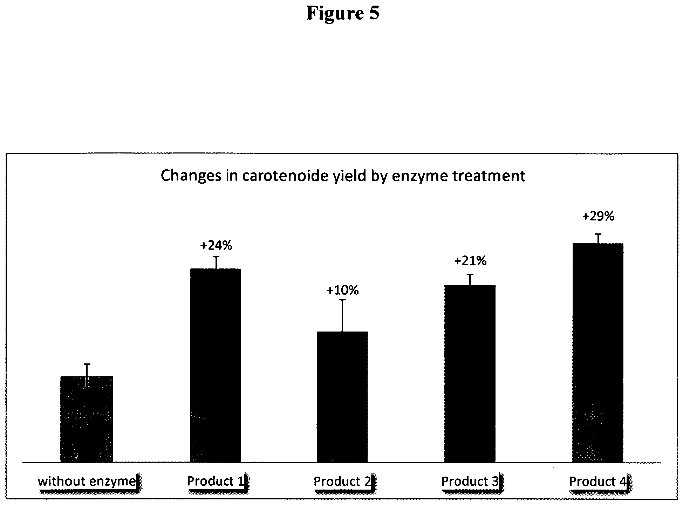

Fig 5 is a diagram showing the effect of mannanase enzyme on cell disruption and carotenoid extraction of *Haematococcus pluvialis*

### DETAILED DESCRIPTION OF THIS DISCLOSURE

The enzymes of the present invention may be used to improve the extractability of an algae cellular composition from algae cells belonging to the genus *Haematococcus.* The recovery of a carotenoids like astaxanthin from the hard-cell-walled haematocysts of *Haematococcus* is one of the objects of the present disclosure.

Embodiment of the present disclosure are related to methods for producing a cell component of an algae cells, for examples natural cell components like pigments or lipids as well as products produced by the algae cells e.g. after a genetic modification of the cells. The methods according to the present disclosure may be methods for producing carotenoids, biofuels or oil products, as well as the production of pharmaceutical active components.

For example, in some embodiments, enzymes according to the present disclosure may be added to algal cells suspended in solutions to degrade the algal cell walls and release their content, whereas in some embodiments, nucleic acid molecules encoding such enzymes may be introduced into the algal cells to express the enzymes therein, so that these enzymes can degrade the algal cell walls and/or algae cyst cell walls from within.

In some embodiments, nucleic acids encoding enzymes capable of catalyzing algae cell degradation may be introduced into algal cells to express the enzymes in those cells and to degrade their cell walls, while enzymes may also added to or mixed with the cells to further promote the cell degradation.

It is an advantage of the methods and enzyme compositions according to the present disclosure that after the enzymatic degradation of the algae cells additional cell degradation methods by chemical and/or physical treatment are not needed any more or can be reduced to a minimum on time.

However, in some embodiments the methods according to the present disclosure comprises an additional non-enzymatic treatment step like heating, sonication, mechanical lysis, osmotic shock, expression of an autolysis gene, exposure to pH above 8 and exposure to pH below 6.

Disclosed herein are methods of improving the extractability of cell components of algae using enzymes and/or mixtures of enzymes according to the present disclosure. In some embodiments, methods for improving the extractability of a cellular composition from algae according to the present disclosure comprise the following steps:
a) Cultivating and growing algae cells to a desired algae biomass
b) Subjecting the algae biomass to an enzyme composition comprising one or more enzymes capable to degrading the algae cells
c) Extracting the cellular composition from the algae biomass

In advantageous embodiments, the present disclosure relates to methods for producing an algae cell component comprising the steps of:
a) culturing and growing algae in a liquid medium to a desired algae biomass,
b) harvesting the algae,
c) enzymatic treatment of the algae with an enzyme composition comprising a mannanase and a lipase,
d) isolating the cell component from the algae biomass.

In some embodiments the cellular composition may be pigments like carotenoids, carbohydrates like starch, lipids like poly unsaturated fatty acids (PUFA), proteins, amino acids, vitamins and mineral nutrients. In an advantageous embodiment, the cellular composition is a carotenoid like astaxanthin. In yet another embodiment of the disclosure, chlorophylls and carotenoids are isolated from the neutral lipids.

In an advantageous embodiment, the cellular composition is a carotenoid. As used herein the term "carotenoid" stands for the chemical compound as such as well as for a pigment of the appropriate dye, if not otherwise stated. For example, in case of astaxanthin the chemical compound 3,3'-dihydroxy-beta,beta-carotene-4,4'-dione in form of the (3S,3'S)- isomer is meant as well as the dye as a pigment. Carotenoids are colored lipid-soluble compounds that can be found in higher plants and algae, as well as in nonphotosynthetic organisms like animals (although they are not able to synthesize carotenoids), fungi, and bacteria. Carotenoids are responsible for the red, orange and, yellow colors of plant leaves, fruits, and flowers, as well as for the color of feathers, crustacean shells, fish flesh and skin, etc. (Gudin 2003; Johnson and Schroeder 1995; Negro and Garrido-Fernández 2000). The chemical structure of the more than 600 different carotenoids is derived from a 40-carbon polyene chain, which can be considered as the backbone of the molecule. The polyene system gives carotenoids their distinctive molecular structure, their chemical properties, and their light-absorbing characteristics. This chain may be terminated by cyclic groups (rings) and can be complemented with oxygen-containing functional groups. The hydrocarbon carotenoids are named carotenes, whereas oxygenated derivatives are known as xanthophylls. In the latter, oxygen can be present as OH groups (as in lutein), as oxi-groups (as in cantaxanthin), or in a combination of both (as in astaxanthin; Higuera- Ciapara et al. 2006).

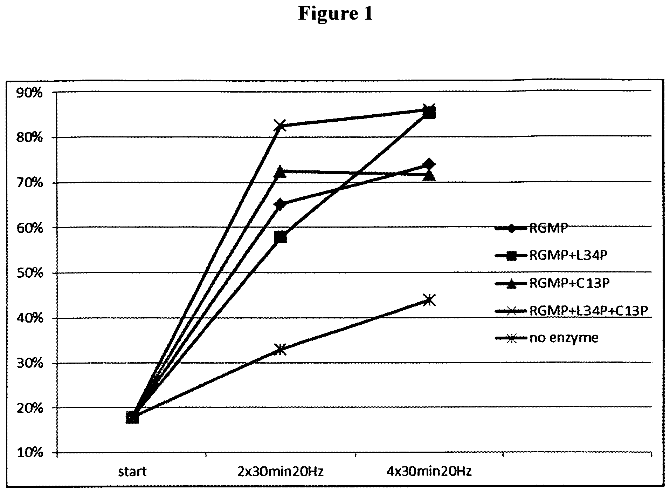

As mentioned, the astaxanthin molecule has two asymmetric carbons located at the 3 and 3" positions of the benzenoid rings on either end of the molecule. Different enantiomers of the molecule result from the exact way that these hydroxyl groups (-OH) are attached to the carbon atoms at these centers of asymmetry (Figure 1). If the hydroxyl group is attached so that it projects above the plane of the molecule it is said to be in the R configuration and when the hydroxyl group is attached to project below the plane of the molecule it is said to be in the S configuration. Thus the three possible enantiomers are designated R,R', S,S' and R,S' (meso). In an advantageous embodiment the produced/extracted carotenoid is astaxanthin in form of the (3 S, 3 'S) isomer.

Free astaxanthin is the form in which the 3 and 3' hydroxyl groups are not bonded, an astaxanthin monoester has one fatty acid bonded to a 3 position, and an astaxanthin diester has a fatty acid bonded to each of the 3 positions. Free astaxanthin and its mono- and diesters for example form algae of the genus *Haematococcus* have optically pure (3S,3'S)-chirality (Renstrom et al., 1981). The monoesters and diesters are relatively non-polar, whereas free carotenoids are relatively polar. The carotenoid fraction of *Haematococcus* algae contains about 70% monoesters of astaxanthin, 10% diesters of astaxanthin, 5% free astaxanthin, with the remainder consisting of β-carotene, canthaxanthin, and lutein.

In a first step, the algae are cultured and grown in a liquid medium to a desired algae biomass. The algae are cultured the production of cellular composition {e.g., lipids, fatty acids, aldehydes, alcohols, alkanes, carotenoids etc.). The former type of culture is conducted on a small scale and initially, at least, under conditions in which the starting microorganism can grow. For example, if the starting microorganism is a photoautotroph the initial culture is conducted in the presence of light. The culture conditions can be changed if the microorganism is evolved or engineered to grow independently of light. The algae can be cultured in bioreactors or ponds.

Microalgae, in particular microalgae of the genus *Haematococcus* are expensive to produce, although many efforts are under way addressed to achieve cost-efficient modes for mass cultivation of these organisms. Different systems have been designed for the growth and handling of microalgae on a large scale (Borowitzka 1999; Gudin and Chaumont 1980; Molina-Grima et al. 1999; Pulz 2001; Richmond 2004; Tredici 2004; Weissman et al. 1988). Within the open systems, the best choice seems to be the open shallow pond, made of leveled raceways 2-10 m wide and 15-30 cm deep, running as simple loops or as meandering systems. Each unit covers an area of several hundred to a few thousand square meters. Turbulence is usually provided by rotating paddle wheels, which create a flow of the algal suspensions along the channels at a rate of 0.2-0.5 m s⁻¹.

The algae according to the present disclosure are green algae with the genus *Haematococcus,* in particular algae of the species *Haematococcus pluvialis* (H. pluvialis), *Haematococcus lacustris* (H. lacustris), *Haematococcus capensis* (H. capensis), *Haematococcus droebakensi* (H. droebakensi), and *Haematococcus zimbabwiensis* (H. zimbabwiensis). In an advantegeous embodiment, the algae are of the species *Haematococcus pluvialis.*

In the present disclosure, green algae described above that contain astaxanthin are used. When green algae are subjected to stresses from the environment, such as nutrient deprivation or the presence of oxides, the green algae accumulate astaxanthin within the cells and become resting spores. The shift to this resting state is referred to as encystment. In this specification, encystment refers to any state from the beginning of the resting state where accumulation of astaxanthin starts, to the completely encysted state where the cells become resting spores. In order to increase the astaxanthin content, it is preferable to use green algae in which encystment has progressed as far as possible and which has accumulated a large amount of astaxanthin. It should be noted that "cultivating encysted green algae" as used herein also includes the process of inoculating green algae containing astaxanthin that has been grown in a nutrient medium, after the algae has reached the encysted state. In the present specification, "green algae" are also intended to include encysted green algae.

Haematococcus cultures are very sensitive to contamination by other microorganisms as well as to extreme environmental conditions (high and low temperature or light). *Haematococcus* haematocysts contain 1.5-3% of the dry biomass after this reddening phase. Harvesting of *Haematococcus* may be accomplished by settling and subsequent centrifugation, as the big and sticky haematocysts sediment easily. Then, this biomass can be dried and cracked to fracture the thick and hard cell wall of the cysts, thus ensuring maximum bioavailability. This cracking step is one of the objections of the present disclosure.

There is no particular limitation on the medium used to cultivate the green algae. Generally, a medium is used that contains nitrogen, inorganic salts of trace metal (e.g., phosphorous, potassium, magnesium, and iron), vitamins (e.g., thiamine), and the like, which are essential to growth. For example, media such as the VT medium, C medium, MC medium, MBM medium, and MDM medium (see Sorui Kenkyuho, ed. by Mitsuo Chihara and Kazutoshi Nishizawa, Kyoritsu Shuppan (1979)), the OHM medium (see Fabregas et al., J. Biotech., Vol. 89, pp. 65-71 (2001)), the BG-11 medium, and modifications thereof may be used. In the present invention, it is preferable to use an autotrophic medium that is substantially free from organic carbon source so that contamination by bacteria can be prevented.

These media may be selected depending on their purposes, such as growth, or encystment. For example, for growth of the green algae, a medium having a large amount of components serving as a nitrogen source is used (rich medium: containing at least 0.15 g/L expressed in terms of nitrogen). For encystment, a medium having a small amount of components serving as a nitrogen source is used (encystment medium: containing less than 0.02 g/L expressed in terms of nitrogen). Alternatively, a medium containing a nitrogen source at an intermediate concentration between these media may be used (low nutrient medium: containing at least 0.02 g/L and less than 0.15 g/L expressed in terms of nitrogen).

The nitrogen source concentration, phosphorous concentration, and other properties of the medium can be determined depending on the amount of the green algae to be inoculated. For example, when a green algae count in the order of 10⁵ is inoculated in a low nutrient medium, the green algae would grow to a certain extent, but the growth may stop soon because the amount of the nitrogen source is too small. Such a low nutrient medium is suitable for performing growth and encystment continuously in a single step (in a batch manner), as described later. Furthermore, by adjusting the N/P mole ratio to value from 10 through 30, preferably 15 through 25, the green alga can be encysted.

There is no particular limitation on the apparatus for cultivating the green algae, as long as the apparatus is capable of supplying carbon dioxide and irradiating a culture suspension with light. For example, in the case of a small-scale culture, a flat culture flask may be preferably used. In the case of a large-scale culture, a culture tank that is constituted by a transparent plate made of glass, plastic, or the like and that is equipped with an irradiation apparatus and an agitator, if necessary, may be used. Examples of such a culture tank include a plate culture tank, a tube-type culture tank, an airdome-type culture tank, and a hollow cylinder-type culture tank. However, also open or closed ponds can be used for growing the algae, and seawater as a natural culture medium.

There is no particular limitation on the culture conditions, and a temperature, a pH, and the like as generally employed for cultivation of algae can be used. The green algae are cultivated at, for example, 15 to 35 °C, and preferably 20 to 25 °C. It is preferable that the pH is maintained at 6 to 8 throughout the cultivation period. Carbon dioxide is supplied by bubbling a gas containing carbon dioxide at a concentration of 1 to 3 v/v% at a rate of 0.2 to 2 wm, for example. When a plate culture tank is used, the culture suspension is stirred by supplying carbon dioxide, so that the green algae can be uniformly irradiated with light.

In the case where the green algae count for inoculation is even higher, the rich medium can be employed to perform the above-described cultivation.

In this manner, the composition of the medium can be determined in consideration of various conditions. It should be noted that the medium preferably used in the present invention, i.e., an autotrophic medium, is nearly free from an organic carbon source such as acetic acid or glucose, so that contamination by bacteria hardly occurs even in long-term cultivation.

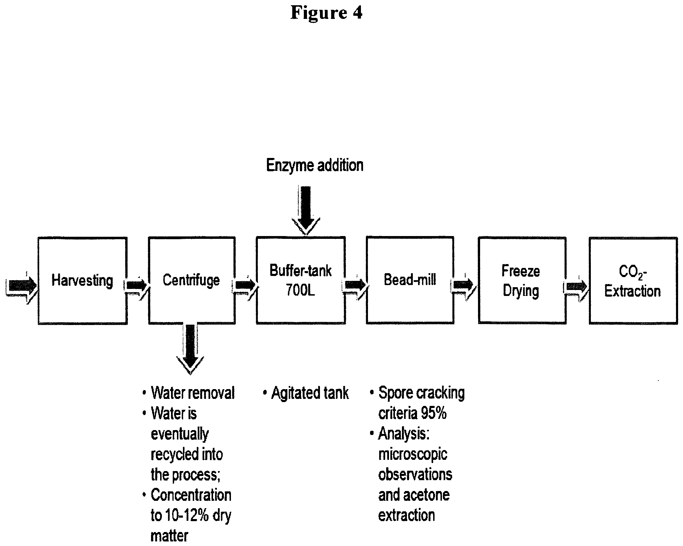

In a second step, the algae cells are harvested and optionally centrifuged to reduce the water content. The harvested biomass can be transferred in a buffer tank (see Fig.4). In an advantageous embodiment the algae biomass is then treated with an enzyme composition.

In an advantage embodiment of the present disclosure the enzyme composition comprises a hemicellulase. "Hemicellulase" refers to a protein that catalyzes the hydrolysis of hemicellulose, such as that found in lignocellulosic materials. Hemicellulose is a complex polymer, and its composition often varies widely from organism to organism and from one tissue type to another. Hemicelluloses include a variety of compounds, such as xylans, arabinoxylans, xyloglucans, mannans, glucomannans, and galactomannans. Hemicellulose can also contain glucan, which is a general term for beta-linked glucose residues. In general, a main component of hemicellulose is beta-1,4-linked xylose, a five carbon sugar. However, this xylose is often branched as beta- 1,3 linkages or beta- 1,2 linkages, and can be substituted with linkages to arabinose, galactose, mannose, glucuronic acid, or by esterification to acetic acid. The composition, nature of substitution, and degree of branching of hemicellulose is very different in dicotyledonous plants (dicots, i.e., plant whose seeds have two cotyledons or seed leaves such as lima beans, peanuts, almonds, peas, kidney beans) as compared to monocotyledonous plants (monocots; i.e., plants having a single cotyledon or seed leaf such as corn, wheat, rice, grasses, barley). In dicots, hemicellulose is comprised mainly of xyloglucans that are 1,4- beta-linked glucose chains with 1 ,6-alpha- linked xylosyl side chains. In monocots, including most grain crops, the principal components of hemicellulose are heteroxylans. These are primarily comprised of 1,4-beta-linked xylose backbone polymers with 1,2- or 1,3-beta linkages to arabinose, galactose and mannose as well as xylose modified by ester- linked acetic acids. Also present are branched beta glucans comprised of 1,3- and 1,4- beta-linked glucosyl chains. In monocots, cellulose, heteroxylans and beta glucans are present in roughly equal amounts, each comprising about 15-25% of the dry matter of cell walls. Hemicellulolytic enzymes, i.e. hemicellulases, include both endo-acting and exo- acting enzymes, such as xylanases, [beta]-xylosidases. galactanases, [alpha]-galactosidases, [beta]-galactosidases, endo-arabinases, arabinofuranosidases, mannanases, [beta]-mannosidases. Hemicellulases also include the accessory enzymes, such as acetylesterases, ferulic acid esterases, and coumaric acid esterases. Among these, xylanases and acetyl xylan esterases cleave the xylan and acetyl side chains of xylan and the remaining xylo-oligomers are unsubstituted and can thus be hydrolysed with [beta]-xylosidase only. In addition, several less known side activities have been found in enzyme preparations which hydrolyze hemicellulose. Accordingly, xylanases, acetylesterases and [beta]-xylosidases are examples of hemicellulases.

In an advantage embodiment of the present disclosure the enzyme composition comprises a mannanase or a functional equivalent thereof. The term "mannanase" refers to any enzyme capable of hydrolyzing polyose chains that are composed of mannose units (mannopolymers or polymannoses). "Mannanase" therefore comprises both endomannanases and exomannanases which cleave mannopolymers internally or from the terminal ends of the polymer, respectively. In advantageous embodiments a 1,4-beta- mannanase is used, preferably a mannanase with endo-1,4-beta-mannanase main activity.

Endo-β-1,4-D-mannanase (β-mannanase; EC 3.2.1.78) catalyses the random hydrolysis of manno-glycosidic bonds in mannan-based polysaccharides. Most β-mannanases, degrade oligosaccharides down to DP4 (Biely and Tenkanen (1998) Enzymology of hemicellulose degradation, pages 25-47. In Harman and Kubiceck (ed) Trichoderma and Gliocladium, vol.2, Taylor and Francis Ltd. London), however, residual activity has been demonstrated on mannotriose, indicating at least four subsites for mannose binding on the protein. The main end products of hydrolysis are often mannobiose and mannotriose, although significant amounts of mannose are also produced. Some β-mannanases are able to degrade crystalline mannan. In addition to hydrolysis, several β-mannanases, including β-mannanase from *Trichoderma reesei*, have been shown to form transglycosylation products with either mannose or mannobiose as glycosidic bond acceptor.

β-mannanases have been isolated from a wide range of organisms including bacteria, fungi, plants and animals. Although mostly extracellular, some β-mannanases, appear to be cell-associated. Their expression is often induced by growth on mannan or galactomannan, however, β-mannanase, from *T*. *reesei* can also be induced by cellulose, while its expression is suppressed by glucose and other monosaccharides. Frequently multiple mannanases with different isoelectric points are found in the same organism, representing products from different genes or different products from the same gene, respectively.

In one embodiment of the present disclosure, the enzyme composition shows the mannanase enzyme activity as the main activity. In this case, even though the enzyme composition comprises other enzymes as a mannanase or a functional equivalent thereof, the mannanase activity is the main activity in the enzyme mix. For example if the enzyme composition comprises a mannanase and a cellulase, the cellulase shows only a side activity in the enzyme mixture and could be seen as a contamination of the enzyme composition. In other words, in an advantageous embodiment of the disclosure the enzyme composition shows only a small cellulase activity and a high mannanase activity.

In a further advantageous embodiment of the disclosure the enzyme composition comprises another enzyme capable to degrade the algae cells, in particular selected from the group consisting of, but not limited to other hemicellulases, alpha-galactosidases, beta-galactosidases, lactases, laminarinase, glucanases, beta-glucanases, endo-beta-1,4-glucanases, cellulases, xylosidases, xylanases, xyloglucanases, xylan acetyl-esterases, galactanases, exo-mannanases, pectinases, pectin lyases, pectinesterases, polygalacturonases, arabinases, rhamnogalacturonases, laccases, reductases, oxidases, phenoloxidases, ligninases, proteases, amylases, phosphatases, lipolytic enzymes, esterases, cutinases and/or others. In an advantageous embodiment, the glucanase is an endo-1, 3(4)-beta glucanase.

In a preferred embodiment, the enzyme composition comprises a laminarinase. Laminarinase is capable of hydrolysing laminarin or callose. To date, two laminarinases have been identified, endo-β-1,3(4)-glucanase (EC 3.2.1.6) and endo-β-1,3-glucanase (EC 3.2.1.39); endo-β-1,3(4)-glucanase (EC 3.2.1.6) is capable of hydrolysing both β-1,3- and β-1,4-glycosidic bonds, while endo-β-1,3-glucanase (EC 3.2.1.39) is capable of hydrolysing mainly β-1,3-glycosidic bonds (Boeckmann et al., 2003; Terra and Ferreira, 1994). Laminarinase may also work synergistically with cellulases such as endo-β-1,4-glucanase to hydrolyse structural polysaccharides within the plant cell wall (Mansfield et al., 1999).

In a preferred embodiment, the enzyme composition comprises a lipase. A lipase is an enzyme that catalyzes the hydrolysis of ester bonds in water-insoluble, lipid substrates. Lipases catalyze the hydrolysis of lipids into glycerols and fatty acids.

In another embodiment the enzyme composition comprises at least a further enzyme in addition to the above mentioned enzymes like a protease, cellulase, pectinase and/or a glucanase, or mixture thereof. In one embodiment, the glucanase is an endo-1,3(4)-beta glucanase.

"Pectinase" according to the present disclosure refers to enzymes, such as pectinlyase, pectinesterases and polygalacturonase and combinations thereof which break down pectin.

A "Protease" according to the present disclosure is any enzyme that conducts proteolysis, that is, begins protein catabolism by hydrolysis of the peptide bonds that link amino acids together in the polypeptide chain forming the protein.

"Cellulase" according to the present disclosure is any enzymes that catalyze cellulolysis (i.e. the hydrolysis) of cellulose.

In an advantageous embodiment of the disclosure the enzyme composition comprises a mannanase as main activity. In a further embodiment of the disclosure the enzyme composition comprises a lipase as main activity. In another advantageous embodiment the enzyme composition comprises a laminarinase and/or a 1,3(4) beta-glucanase as main activity.

In an advantageous embodiment of the disclosure the enzyme composition comprises a mannanase and a lipase as main activities. In a further advantageous embodiment, the enzyme composition comprises a mannanase, a lipase and a cellulase as main activities. In another advantageous embodiment of the disclosure the enzyme composition comprises a mannanase and a protease or/and a mannanase and a cellulase as main activities. In another advantageous embodiment of the disclosure the enzyme composition comprises a mannanase, a laminarinase and a lipase as main activities. In another advantageous embodiment of the disclosure the enzyme composition comprises a mannanase, a lipase, a laminarinase and/or a 1,3(4) beta-glucanase as main activities. In a further advantageous embodiment the enzyme composition comprises a mannanase, a protease and a cellulase as main activities. In a further advantageous embodiment the enzyme composition comprises mannanase, a pectinase and a cellulase as main activities. In another advantageous embodiment the enzyme composition comprises a lipase and a laminarinase and/or a 1,3(4) beta-glucanase as main activities. In another advantageous embodiment the enzyme composition comprises a mannanase and a laminarinase and/or a 1,3(4) beta-glucanase as main activities.

The enzyme combination comprised in the enzyme composition for extracting astaxanthin from microalgae like green algae, preferably from encysted green algae, preferably with the genus *Haematococcus* preferably from *Haematococcus pluvialis* is the combination of a mannanase, a pectinase and a cellulase, preferably the combination of a mannanase, a laminarinase and a lipase, more preferably the combination of a mannanase and a lipase.

In another aspect, the disclosure pertains to methods for producing an algae cell component, in particular a carotenoid by using algae belonging to the genus *Haematococcus* comprising the steps of:
a) culturing the algae in a liquid medium,
b) harvesting the algae,
c) enzymatic treatment of the algae cells or parts thereof with with an enzyme composition comprising a mannanase and/or a lipases and/or a laminarinase,
d) isolating the cell component

As used herein the term "isolation" as used in the present disclosure refers to a process or means that is suitable to obtain an algae cell component form the cracked algae like extraction or centrifugation. The isolation of algae cell component for further use or analysis can also carried out by known extraction procedures. In particular, the cells may be harvested by centrifugation, for example at 1900 x g for 3 minutes, and washed once or twice in water. The cell pellet that is obtained by centrifugation is broken with mortar and pestle with the aid of aluminium powder and then resuspended in a suitable organic solvent, for instance in acetone or methanol and the carotenoid extract is separated from the cell debris by centrifugation at 1900 x g, saponificated with a mixture of the same volumes of 2 percent (w/v) solution of KOH in methanol and diethyl ether, then the supernatant is evaporated under N2 and the pellet is resuspended in acetone, centrifuged and analyzed by HPLC. The process is carried out at a temperature between 0 Degrees C and 40 °C particularly 5 °C and 35 °C more particularly 10 °C and 30 °C preferably at room temperature, preferably in the dark and the carotenoid extract is kept at a temperature between -20 °C and 25 °C more particularly -20 and 4 °C preferably at -20 °C. Optionally, the samples obtained can be collected and centrifuged once more to separate undesired particles from the cells or extracts. The supernatant can be used for further spectrophotometric analysis, as mentioned above, for HPLC or other technologies concerning analysis of carotenoids or cells containing same, such as thin layer chromatography, for example using Kiesel gel plates, gas chromatography or magnetic resonance chromatography.

In general, an algae cell component, in particular a carotenoid produced with a method according to the present disclosure can be isolated and/or extracted by methods known in the art. For example the carotenoids can be isolated by extraction from the microorganism or parts therefrom, such as cell debris or physically pressed cells, using an organic solvent as mentioned above.

As regards analysis by HPLC reverse phase HPLC can be used according to known procedures. In particular, a Waters Spherisorb S5 ODS 18 4.6 x 250 mm cartridge column can be used and a solvent linear gradient from 100 percent solvent A (acetonitrile: methanol: 0.1 M Tris-HCl, pH 8.0 [84: 2: 14]) to 100 percent solvent B (methanol: ethyl acetate [68: 32]) for 15 inin, followed by 3 min of solvent B, which is pumped by using a Dual Dispensity system with a flow rate of 1.2 ml min'1 from which carotenoid pigments can be eluted. The pigments can be detected by using a photodiode-array detector (Waters 2996) at 440 run. The concentration of individual carotenoids is determined using standard curves of purified pigments at known concentrations. Astaxanthin can be determined also by measuring the absorbance at 477 nm using an extinction coefficient of 2100. It is known from the literature, that the obtained carotenoid astaxanthin is achievable in the pure form of the (3S,3'S) isomer.

In some embodiments, the methods according to the present disclosure comprises an additional non-enzymatic treatment step selected from the group consisting of heating, sonication, mechanical lysis, osmotic shock, expression of an autolysis gene, exposure to pH above 8 and exposure to pH below 6. In particular, the disruption of the algae cell walls can suitably be made by one or more methods within the group consisting of ultra-sonication, liquid shear disruption, bead milling, high pressure pressing, freeze-thawing, freeze-pressing, hydrolysation, and virus degradation.

In an advantage embodiment, the non-enzymatic treatment step is a mechanical lysis, preferably milling, in particular bead-milling.

This non-enzymatic treatment step can be carried out before and/or after the enzymatic treatment. In some embodiments, the non-enzymatic treatment step is carried out before the enzymatic treatment step to disrupt the algae cell walls. The following enzymatic treatment of the non-enzymatic treated algae biomass comprising non disrupted algae cells, parts of the algae cell like compartments and/or cell walls with an enzyme composition according to the present disclosure increase the yield of the desired cell components like carotenoids. In one embodiment an increase of 8% in the yield of carotenoids from *Haematococcus pluvialis* after an acetone extraction was obtained. In other embodiments, the non-enzymatic treatment step is carried out after the enzymatic treatment step. The enzymatic treatment with an enzyme composition according to the present disclosure disrupt the algae cell walls, the following energy consuming mechanical lysis e.g. via milling can be reduced in time significantly.

The use of the enzyme compositions according to the present disclosure shows a clear effect on algae cell disruption in lab scale testing as well as in pilot scale. The use results in a significant decrease of the processing time. Furthermore, the yield of the produced algae cell component is increased.

Therefore, the enzymes and enzyme compositions of the present disclosure may be used for an improved release of the contents of an algae cell. In some embodiments, contacting or mixing the algae cells with the enzymes of the present disclosure will degrade the cell walls, resulting in cell lysis and release of the cellular contents. For example, the enzymes of the present disclosure may be used to degrade the cell walls of micro- and macro algal cells in order to release the materials contained within the algal cells. In some embodiments, such materials may include, without limitation, carotenoids, alcohols and oils. The alcohols and oils so released can be further processed to produce bio-diesel, jet fuels, as well as other economically important bio-products.

The enzymes and enzyme compositions of the present disclosure may be used alone, or in combination with other enzymes, chemicals or biological materials. The enzymes of the present disclosure may be used for *in vitro* applications in which the enzymes or mixtures thereof are added to or mixed with the appropriate substrates to catalyze the desired reactions.

Additionally, the enzymes of the present disclosure may be used for *in vivo* applications in which nucleic acid molecules encoding the enzymes are introduced into algal cells and are expressed therein to produce the enzymes and catalyze the desired reactions within the cells.

For example, in some embodiments, enzymes capable of promoting cell wall degradation may be added to algal cells suspended in solutions to degrade the algal cell walls and release their content, whereas in some embodiments, nucleic acid molecules encoding such enzymes may be introduced into the algal cells to express the enzymes therein, so that these enzymes can degrade the algal cell walls from within. Some embodiments may combine the *in vitro* applications with the *in vivo* applications. For example, nucleic acids encoding enzymes capable of catalyzing cell wall degradation may be introduced into algal cells to express the enzymes in those cells and to degrade their cell walls, while enzymes may also added to or mixed with the cells to further promote the cell wall degradation. In some embodiments, the enzymes used for *in vitro* applications may be different from the enzymes used for *in vivo* applications. For example, an enzyme with the mannanase activity may be mixed with the cells, while an enzyme with the protease activity is expressed within the cells.

In one aspect, the present disclosure includes proteins isolated from, or derived from the knowledge of enzymes from a filamentous fungus such as *Aspergillum, Trichoderma* or a mutant or other derivative thereof. For example, if a filamentous fungus was cultivated on an algae species, the proteins, preferably proteins with enzymatic activity of the filamentous fungus can be isolated and used for the degradation of the same algae species in a production process.

Figure 1 shows an example for the use of different enzyme compositions according to the present disclosure for the extraction of carotenoids as a typical cell component. The enzymes compositions comprise one or more of the following commercially available enzymes:
a) RGMP = Rohalase GMP from AB Enzymes (comprises mannanase activity as main activity)
b) L34P = Lipomod 34P from Biocatalysts, Ltd. (comprises lipase / esterase activity as main activity)
c) C13P = Cellulase 13P from Biocatalysts Ltd. (comprises cellulase activity as main activity)

The treatment of the algae biomass with the enzyme compositions shows better carotenoids extraction (in %) in contrast to the control batch where no enzyme was added to the algae.

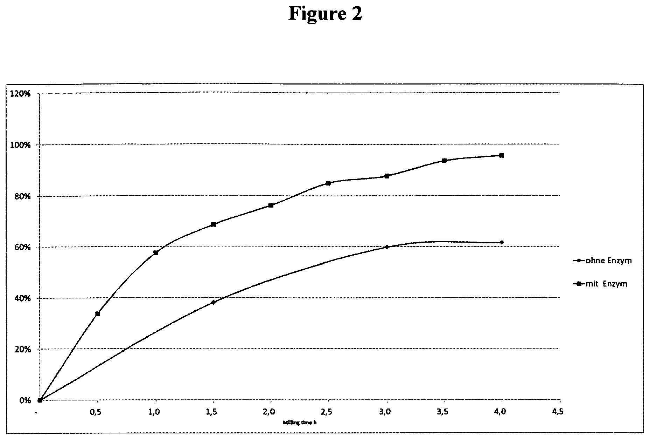

Figure 2 shows also the improved extraction of carotenoids as a typical cell component after milling the mannanase treated algae biomass. After the treatment of the algae biomass with the mannanases, the following milling time to extract the carotenoids is reduced. The enzyme was added after the algae cells were cultivated and growth to the desired algae biomass and harvested via centrifugation. After subjecting the algae biomass to the enzyme composition the biomass was milled to extract the carotenoids.

However, in further examples it was shown that no additional cell disruption methods were needed after the treatment of the algae biomass with enzyme compositions according to the present disclosure.

The methods according to the present disclosure can be also used in the production of biofuels like biodiesel and bioethanol, and/or in the production of animal feed.

The extraction of algae lipids for the production of algae biofuels is one example for the use of the extraction methods according to the present disclosure. Furthermore, the enzymatically degradation of the algae cell walls increases the availability of proteins and amino acids, lipids as PUFA's and/or carotenoids like astaxanthin, when they are used as animal feed. Due to the high extraction rate when using the methods according to the present disclosure, algae biomass can be used as an alternative to fish meal in aquaculture farming.

Unless defined otherwise herein, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

The headings provided herein are not limitations of the various aspects or embodiments of this disclosure which can be had by reference to the specification as a whole. Accordingly, the terms defined immediately below are more fully defined by reference to the specification as a whole.

The following methods and examples are offered for illustrative purposes only, and are not intended to limit the scope of the present disclosure in any way.

### Methods and Examples

In the following examples, materials and methods of the present disclosure are provided. It should be understood that these examples are for illustrative purpose only and are not to be construed as limiting this disclosure in any manner.

### Example 1:

Effect of different enzyme on the extraction of carotenoids out of *Haematococcus pluvialis* The algae were grown in 10L shaking flasks and separated using a centrifuge.

The following steps were carried out to measure the extraction rate of carotenoids after the enzymatic treatment:

| | |
|---|---|
| Algae suspension | 10% in NaCl 0,9% |
| Enzyme | 10% in NaCl 0,9% |
| Enzyme Solution | NaCl 0,9% |
| batch | 500µl Algen + 1/100 (5µl) Enzyme Incubation overnight (approx. 20h)at |
| duration/temp | 35°C with shaking of 500rpm-followed by milling , extraction and spectrometric measurement |

**milling (Retsch-Mill)**

| | |
|---|---|
| Volume | 500µl |
| Glass beads | 250mg |
| Milling duration | 2x and 4x 30min |
| Frequence | 20 Hz |

**Extraction**

| | |
|---|---|
| Total Volume before Extraction | 100µl |
| Extractions means | Aceton |
| Adding Extractions means | 900µl |
| correlate % | 90% |
| Centrifugation | 2min 2000xg |

s

**Measurement**

| | |
|---|---|
| Dilution to Measurement | 1 to 25 |
| with | Aceton |
| Wave lenghth | 474nm |
| Analyzer | Tecan M1000 |
| Surface Plate | Eppendorf PP |

### Controls

### "untreated"

### "mill"

Compared to the untreated algae cells the degree of cell disruption was significantly increased when using the enzyme composition according to the present disclosure (see Table 1, Fig.1).

**Table 1**

| | start | 2x30min20Hz4x30min20Hz | | |
|---|---|---|---|---|
| RGMP | **18%** | **65%** | **74%** | |
| RGMP+L34P | **18%** | **58%** | **85%** | |
| RGMP+C13P | **18%** | **72%** | **72%** | |
| RGMP+L34P+C13P | **18%** | **83%** | **86%** | |
| no enzyme | **18%** | **33%** | **44%** | |
| c.mill | | | | **100%** |

### Example 2:

### Extraction of carotenoids from Haematococcus pluvialis

The following steps were carried out to measure the extraction rate of carotenoids after the enzymatic treatment:
- 10L Haematococcus suspension with 6,6% (dm) algae cells
- Enzyme added: 50g RGMP in 10L Algaesuspension 0,5% (w/v)
- Pre-incubation with enzyme for 2hours
- Milling in a Netzsch Mill LME4 with a throughput of 150L/h
- Samples of the enzyme treated algae where taken every 30 minutes

The degree of cells disrupted was measured by aceton extraction with 90% acetone, as described in Example 1.

Compared to the untreated algae cells the degree of cell disruption was significantly increased when using the enzyme composition according to the present disclosure (see Table 2, Fig.2).

**Table 2**

| h | with Enzyme | cell disruption % |
|---|---|---|
| 0 | - | |
| | | |
| 0,5 | 0,37 | 34% |
| | | |
| 1,0 | 0,64 | 58% |
| | | |
| 1,5 | 0,76 | 68% |
| | | |
| 2,0 | 0,84 | 76% |
| | | |
| 2,5 | 0,94 | 85% |
| | | |
| 3,0 | 0,97 | 88% |
| | | |
| 3,5 | 1,03 | 94% |
| | | |
| 4,0 | 1,06 | 96% |
| | | |
| h | without Enzyme | |
| - | 0,00 | 0% |
| 1,5 | 0,42 | 38% |
| 3 | 0,66 | 60% |
| 4 | 0,68 | 62% |
| | | |
| | max | |
| | 1,10 | 100% |

### Example 3:

### Effect of Laminarinase and Lipase to improve extraction of Carotenoids from Haematococcus pluvialis

**Table 3**

| | | | |
|---|---|---|---|
| end-1,4-β Mannanase | Dillution before application: 1:2 in water | | Megazyme endo-1,4 β-Mannanase (Bacillus sp.) Catalogue Number: E-BMABS |
| Laminarinase | Dillution before application: 5,64 mg/ml in water | | Laminarinase from Trichoderma sp. |
| | | | Sigma-Aldrich (Catalogue Number: L5272) |
| Lipase from Rizopus niveaus | Dillution before application: 50mg/ml in water | | Lipase from Rhizopus niveus Sigma-Aldrich (Catalogue Number: 62310) |

20 ml aqueous algae suspension (10 - 12,5% dry weight) were mixed with 10ml glass beads (0,3 mm diameter) in shaking flask, followed by the addition of 5ml enzyme solution or water in case of the blank. Enzyme incubation took place at 50°C for 60 minutes on a rotary shaker at 120rpm. The samples were lyophilized overnight and dry matter determined after drying at 105 °C for 24h. Total extractable carotenoids and astaxanthin were determined by acetone extraction following the protocols of Cyanotech or Fuji Chemicals.

The Cyanotech extraction procedure:
The protocol was carried out in low light and temperatures, as carotenoids are very sensitive to light, oxygen and heat. It is recommended that the assay be performed in a darkened room while keeping the temperatures at 5 C if possible.
1) Weigh approximately 25 mg of dried *Haematococcus* algae powder into the 10 ml centrifuge tube.
2) Add 3 grams of glass beads to the tubes.
3) Add 5 ml of DMSO to the centrifuge tubes, place in pre-heated water bath at 45-50 C for 30 minutes. Vortex for 15 seconds every 10 minutes during the incubation.
4) Centrifuge at 3800-4200 rpm for 5 minutes to pellet the cell material.
5) Pipette the supernatant and collect in the 25 ml volumetric flask.
6) Add 5 mls of the acetone to the centrifuge tube, vortex vigorously for 30 seconds. Centrifuge tubes at 3500 rpm for 5 minutes to pellet the cell material. Transfer supernatant to the volumetric flasks.
7) If supernatant still has color, repeat step 6 until centrifuged acetone layer is less than 0.05 absorbency.
8) After all the supernatant is collected in the volumetric flask, bring the volume to 25 ml with acetone.
9) Cap the flask and invert gently to mix. Pipette an aliquot (5-7 mls) into a clean tube and centrifuge for 5 minutes at 3500 rpm to remove any particulate matter which may have carried over in the transfers.
10) Read the maximum absorbency (approx. 471-477 nm) against an acetone blank on the spectrophotometer.
11) If the absorbency is greater than 1.25 it is necessary to dilute the sample with acetone and adjust the calculation for the dilution (absorbency readings are linear between 0.25 and 1.25). A 1:7 dilution (1 part astaxanthin extract to 6 parts of acetone) will typically adjust the final absorbency within the proper range.
12) Proceed to Part 2 for hydrolysis of the carotenoid esters.

**Table 4**

| Enzyme | **% astaxanthin extracted** |
|---|---|
| no Enzyme | 1,004 |
| β-Mannanase | 1,310 |
| Laminarinase | 1,124 |
| Lipase from Rizopus niveaus | 1,156 |

The enzymes mentioned in table 3 were added to the algae biomass. The results are shown in table 4. Due to the treatment of the beta1,4-mannanase the improvement in extractability of *Haematococcus pluvialis* is increased significantly. The application of Laminarinase and Lipase improves the extractability as well.

In a second trial the enzymes were used in combination and the results are shown in table 5. The combination of mannanase with laminarinase and lipase improve the extractability significantly better compared to mannanase treatment alone.

**Table 5**

| **Enzyme** | **% astaxanthin extracted** |
|---|---|
| β-Mannanase/ Laminarinase/ | 1,25 |
| β-mannanase/ Lipase from Rizopus niveaus | 1,25 |
| Laminarinase/ Lipase from Rizopus niveaus | 1,21 |
| β-mannanase/ Laminarinase/ Lipase from Rizopus niveaus | 1,43 |

### Example 4:

### Effect of mannanase enzymes on cell disruption of Haematococcus pluvialis

20 ml algae with 12% dry matter were incubated in a shaking flask containing glass beads with 5 ml of an aqueous enzyme solution. Incubation took place at 50°C, for 1h in an incubation shaker (300ppm). After incubation the extractable carotenoide concentration were measured by acetone extraction according to a standard protocol from Cyanotech or Fuji Chemicals (see example 3).

Different enzyme compositions (Product 1-4) were used for the tests. The mannanase enzyme activities applied per 25ml sample volume were according to the following table:

**Table 6**

| | **Mannanase U/25ml** |
|---|---|
| Product 1 | 585 |
| Product 2 | 44 |
| Product 3 | 314 |
| Product 4 | 854 |

### Enzyme activity was determined using a DNSA Assay:

Substrate for Mannanase test: Galactomannan carob

Substrates were deissolved in buffer to a concentration of 0,8% (w/v)
Buffer: 50 mM NaAcetat, pH 4.5

The Enzymes were diluted in buffer, the right enzyme concentration must be determined for each enzyme. A 90µl substrate and 10 µl enzyme solution were mixed. A blank was measured replacing enzyme solution with water. Incubation for 30 min at 37°C (followed from 10 min at 4°C). Reducing sugars were measured after mixing of 50 µl of the incubated substrate - enzyme mix with 50 µl DNSA-Reagent.

The activity is calculated as Units per µl or mg of enzyme product. 1 unit is defined as the amount of formed reducing ends in µmol per minute. One protease unit is defined as the formation of glycin equivalents per minute.

Figure 1 shows the effect of mannanase enzymes on cell disruption and carotenoid extraction of *Haematococcus pluvialis.* As higher the mannanase concentration in the enzyme preparation as higher is the yield of the extracted carotenoids.

Some embodiments of the present disclosure pertain to:
A) A method for improving the extractability of a cellular composition from algae comprising:
   d) Cultivating and growing algae cells to a desired algae biomass
   e) Subjecting the algae biomass to an enzyme composition comprising one or more enzymes capable to degrading the algae cells
   f) Extracting the cellular composition from the algae biomass, wherein
      - the cellular composition can be selected from the group consisting of pigments, carotenoids, starch, lipids, poly unsaturated fatty acids (PUFA), proteins, vitamins and mineral nutrients.
      - the cellular composition may be astaxanthin.
      - the algae can be *Haematococcus pluvialis* in fresh form or dried form.
         1. the enzyme composition may comprises a mannanase and a lipase.
      - the enzyme composition may comprises further a cellulase.
      - enzyme composition may comprises a mannanase and a protease.
      - the enzyme composition may comprises a mannanase, a pectinase and a cellulase.
      - the enzyme composition may comprises an enzyme mixture with a mannanase main activity.
      - the enzyme composition may comprises the extract of filamentous fungi.
      - the filamentous fungi may be cultivated on algae.
      - the filamentous fungi may be selected from the group of Aspergillum and Trichoderma.
B) A method for producing an algae cell component by using an algae capable of producing an effective amount of the cell component comprising the steps:
   a) culturing the algae in a liquid medium,
   b) harvesting the algae,
   c) disrupting the algae cells by enzymatic treatment with an enzyme composition,
   d) isolating the cell component, wherein
      - the algae cells may be photoautotrophic algae belonging to the Order Volvocales, preferably to the Family Haematococcaceae, more preferably to the Genus Haematococcus, more preferably to the Species Haematococcus pluvialis.
      - the cell component may be a carotenoid, preferably astaxanthin.
      - the enzyme composition may comprises a mannanase, a pectinase and a cellulase.
C) A method for releasing cellular contents comprising contacting an algal cell with an enzyme composition comprising a mannanase, a protease and a cellulase.
D) A method for releasing cellular contents comprising contacting an algal cell with an enzyme composition comprising a mannanase, a pectinase and a cellulase.
E) A composition for degrading green algae cell walls comprising a mannanase, a pectinase and a cellulase.
F) Use of an enzyme composition comprising a mannanase, a pectinase and a cellulase for the extraction of pigments from green algae.

### Additional References

Abo-Shady, A.M., Y.A. 1993. Mohamed, and T. Lasheen. Chemical composition of the cell wall in some green algae species. Biologia Plantarum, 35:629-632.
Afi, L., P. Metzger, C. Largeau, J. Connan, C. Berkaloff, and B. Rousseau. 1996. Bacterial degradation of green microalgae: incubation of Chlorella emersonii and Chlorella vulgaris with pseudomonas oleovorans and flavobacterium aquatile. Org. Geochem., 25:117-130.
Allard, B., M.-N. Rager, and J. Templier. 2002. Occurrence of high molecular weight lipids (c80+) in the trilaminar outer cell walls of some freshwater microalgae. a reappraisal of algaenan structure. Org. Geochem., 33:789-801.
Ban, K., M. Kaieda, T. Matsumoto, A. Kondo and H. Fukuda. 2001. Whole cell biocatalyst for biodiesel fuel production utilizing Rhizopus oryzue cells immobilized within biomass support particles. Biochem. Eng. J., 8: 39-43
Belarbi, E.H., E. Molina, and Y. Chisti. 2000. A process for high yield and scaleable recovery of high purity eicosapentaenoic acid esters from microalgae and fish oil. Enzyme Microb. Technol. 26:516-529.
Borowitzka M.A. and L.J. Borowitzka, editors. 1988. Micro-Algal Biotechnology. Cambridge University Press. Carmen Ceron M., I. Campos, J.F. Sanchez F.G. Acien, E. Molina, and J.M. Fernandez-Sevilla. 2008. Recovery of lutein from microalgae biomass: Development of a process for Scenedesmus almeriensis biomass. J. Agric.Food Chem. 56:11761-11766.
Chisti, Y. 2007. Biodiesel from microalgae. Biotechnology Advances. 25:294-306
Chisti Y. and M. Moo-Young. 1986. Disruption of microbial cells for intracellular products. Enzyme Microb. Technol. 8:194-204.
Doucha J. and K. Livansky. 2008. Influence of processing parameters on disintegration of Chlorella cells in various types of homogenizers. Appl. Microbiol. Biotechnol. 81:431-440.
Eriksen, N.T. 2008. Production of phycocyanin pigment with applications in biology, biotechnology, foods and medicine. Appl. Microbiol. Biotechnol. 80:1-14.
Fleurence J. 1999. The enzymatic degradation of algal cell walls: a useful approach for improving protein accessibility? J. Appl. Phycol. 11:313-314.
Imam S.H., M.J. Buchanan, H.-C. Shin, and W.J. Snell. 1985. The Chlamydomonas cell wall: Characterization of the wall framework. J. Cell Biol. 101:1599-1607.
Logan, B. E. 2004. Extracting hydrogen and electricity from renewable resources. Env. Sci. Technol., pages 160A-167A.
McComb,R.B. and W. D. Yushok. 1958. Colorimetric estimation of D-glucose and 2-deoxy-Dglucose with glucose oxidase. The Biochem. Res. Foundation, pages 417-422. 12 Mendes-Pinto, M.M., M.F.J. Raposo, J. Bowen, A.J. Young, and R. Morais. 2001. Evaluation of different cell disruption processes on encysted cells of Haematococcus pulvialis: effects on astaxanthin recovery and implications for bioavailability. J. Appl. Phycol., 13:19-24.
Molina Grima E., E.H. Belarbi, F.G. Acien Fernandez, A. Robels Medina, and Y. Chisti. 2003. Recovery of microalgal biomass and metabolites: process options and economics. Biotechnol. Adv. 20:491-515.
Richmond A.G., editor. 1986. Handbook of Microalgal Mass Culture. CRC Press, Inc Rittman, B.E., 2008. Opportunities for renewable bioenergy using microorganisms. Biotechnol. Bioeng. 100(2):203-212.
Shelef G. and C.J. Soder, editors. 1980 Algae Biomass; Production and Use. Elsevier/North-Holland Biomedical Press. Amsterdam, The Netherlands.
Versteegh, G.J.M., and P. Blokker. 2004. Resistant macromolecules of extant and fossil microalgae. Phycol. Res. 52:325-339.
Washko M.E., and E. W. Rice. 1961. Determination of glucose by an improved enzymatic procedure. Clinical Chem. 7:542-545.
Wurdack M.E., 1923. Chemical composition of the walls of certain algae. Papers from the Department of Botany, Ohio State University, 141:181-191.
Yatsu L.Y., and T.J. Jacks. 1972. Spherosome membranes. Plant Physiol., 49:937-943.
WO 2008/141757 A1
WO 2009/033071
WO 2011/138620

Effective amounts of enzymes in the enzyme compositions according to the present disclosure:
Mannanase may be added in an amount effective in the range from 0.3x10⁶ - 1.6x10⁶ Units per ton algae biomass.

Protease may be added in an amount effective in the range from 0.002 x10⁶-314x10⁶ Units per ton algae biomass.

Xylanase may be added in an amount effective in the range from 0.16x10⁶ - 460x10⁶ Units per ton algae biomass.
1,3(4) beta glucanase may be added in an amount effective in the range from 0.2x10⁶ - 400x10⁶ Units per ton algae biomass.

Lipase may be added in an amount effective in the range from 0.1x10⁶ - 300x10⁶ Units per ton algae biomass.

Laminarinase may be added in an amount effective in the range from 0.2x10⁶ - 400x10⁶ Units per ton algae biomass.

## Claims

1. A method for improving the extractability of astaxanthin in form of the (3 S, 3 'S) isomer from an algae biomass comprising the steps of:
a) Subjecting the algae biomass to an enzyme composition comprising a mannanase, a laminarinase and a lipase,
b) Extracting astaxanthin in form of the (3 S, 3 'S) isomer from the algae biomass,
wherein the algae belongs to the species *Haematococcus pluvialis.*

2. The method according to claim 1, whereby the enzyme composition comprises further a protease.

3. The method according to any one of claims 1 to 2, whereby the enzyme composition comprises further a cellulase.

4. The method according to any one of claims 1 to 3, whereby the enzyme composition comprises further a pectinase.

## Patentansprüche

1. Verfahren zur Verbesserung der Extrahierbarkeit von Astaxanthin in Form des (3 S, 3 'S) - Isomers aus einer Algen-Biomasse, umfassend die Schritte:
a) Aussetzen der Algen-Biomasse einer Enzymzusammensetzung, die eine Mannanase, eine Laminarinase und eine Lipase umfasst,
b) Extrahieren von Astaxanthin in Form des (3 S, 3 'S) -Isomers aus der Algen-Biomasse,
wobei die Algen der Spezies *Haematococcus pluvialis* angehören

2. Verfahren nach Anspruch 1, wobei die Enzymzusammensetzung ferner eine Protease umfasst.

3. Verfahren nach einem der Ansprüche 1 bis 2, wobei die Enzymzusammensetzung ferner eine Cellulase umfasst.

4. Verfahren nach einem der Ansprüche 1 bis 3, wobei die Enzymzusammensetzung ferner eine Pektinase umfasst.

## Revendications

1. Procédé d'amélioration de l'extractibilité de l'astaxanthine sous la forme de l'isomère (3 S, 3 'S) à partir d'une biomasse d'algues comprenant les étapes suivantes :
a) soumettre la biomasse d'algues à une composition enzymatique comprenant une mannanase, une laminarinase et une lipase,
b) extraire l'astaxanthine sous la forme de l'isomère (3 S, 3 'S) à partir de la biomasse d'algues,
dans lequel les algues appartiennent à l'espèce *Haematococcus pluvialis.*

2. Procédé selon la revendication 1, dans lequel la composition enzymatique comprend en outre une protéase.

3. Procédé selon l'une quelconque des revendications 1 et 2, dans lequel la composition enzymatique comprend en outre une cellulase.

4. Procédé selon l'une quelconque des revendications 1 à 3, dans lequel la composition enzymatique comprend en outre une pectinase.
